# EUROPEAN PATENT APPLICATION

(11) **EP 2 226 073 A1**
(43) Date of publication of application: **08.09.2010**
(21) Application number: 09154090.6
(22) Date of filing: 02.03.2009
(51) Int. Cl.: A61K 31/195, A61K 45/06

(54) **Topical pharmaceutical composition comprising an antiseptic and arginine for wound healing**

(71) Applicant: EURO-CELTIQUE S.A., 1653 Luxembourg (LU)
(72) Inventor: Kämpfer, Heiko, 65558, Langenscheid (DE)
(74) Representative: Bühler, Dirk

(57) **Abstract**

The present invention relates to a pharmaceutical composition for topical application, comprising as active ingredients (a) at least one antiseptic agent and/or at least one agent promoting wound healing, and (b) L-arginine.

## Description

### FIELD OF THE INVENTION

The present invention relates to a topical pharmaceutical dosage forms for the treatment of wounds. More particularly, the invention relates to a pharmaceutical composition for topical application comprising as active ingredients (a) at least one antiseptic agent and/or at least one agent promoting wound healing, and (b) L-arginine.

### BACKGROUND

Cutaneous lesions (wounds) may be caused by mechanical, physical or chemical influences. Thermal or chemical bums primarily result in wound edema and loss of intravascular fluid volume due to increased vascular permeability. In spontaneous wound healing, dead tissue sloughs off as new epithelium begins to cover the injured area. With superficial bums (such as sunburns), regeneration usually occurs rapidly from uninjured epidermal elements, follicles, and glands. In the case of destruction of the epidermis or even much of the dermis (e.g., due to surgical lacerations), re-epithelialization starts from the edges of the wound, the scattered remains of integument, or the remaining dermal appendages. This procedure is relatively slow. Excessive granulation tissue forms before being covered by epithelium. To this end, the wounds generally contract and develop to disfiguring or disabling scars unless proper treatments are employed (reviewed, e.g., in Frank, S. et al. (2002) Kidney Int. 61, 882-888; Rizk, M. et al. (2004) World J. Surg. 28, 301-306).

Wound repair represents a complex, highly coordinated process characterized by temporally and spatially overlapping phases of tissue movements comprising hemorrhage, inflammation, re-epithelialization, granulation tissue formation (proliferation), and the late remodeling phase of repair. Loss of a functional healing process might lead to severe disabilities. Accordingly, chronic, non-healing wound conditions represent a situation of major clinical importance, if nothing else due to the risk of acquiring infections or infestations at the wound sites. Beyond, a series of pathological changes accompanied with several diseases finally leads to severely disturbed wound healing conditions. Among those, the most prominent chronic wound situations are known as decubitus or pressure ulcers, venous ulcers, and diabetic ulcers (reviewed, e.g., in Frank, S. et al. (2002), *supra*; Rizk, M. et al. (2004), *supra*).

The intricate signaling network mediating the intracellular communication during wound repair has still not been fully unraveled. Various factors such as cytokines, growth factors, and tissue inhibitors (e.g., metalloproteinases) have been implicated in these regulatory cascades. Furthermore, increasing evidence has emerged supporting an important role of nitric oxide (NO) in wound repair. NO is released during the oxidation of the amino acid L-arginine and is suggested to participate in multiple cellular processes during wound healing such as vasodilatation, antimicrobial activity, immune response, and anti-platelet aggregation activity. The presence of NO at wound sites appears to primarily facilitate the transition of the wound from the inflammatory phase to the proliferative phase of the healing process (reviewed, e.g., in Wu, G. and Morris, S.M. (1998) J. Biochem. 336, 1-17; Curran, J.N. et al. (2006) Wound Rep. Reg. 14, 376-386).

The precursor molecule L-arginine is one of the most versatile amino acids in the cell. It is particularly required during periods of maximal growth, severe stress, and injury. Among other cellular functions, L-arginine has been shown to promote cellular nitrogen balance and to improve wound healing. Within the wound environment, L-arginine is a substrate for two catabolic pathways: one pathway is catalyzed by nitric oxide synthase (NOS) and results in the production of NO and citrulline, whereas in the other pathway arginase regulates the conversion of L-arginine into ornithine and urea. Ornithine, in turn, is a precursor for the synthesis of proline, a substrate for collagen synthesis, and polyamines which stimulate cell proliferation. Hence, an adequate tissue concentration of L-arginine is essential for efficient wound repair. However, NOS and arginase dynamically compete for L-arginine in the wound milieu in order to assure the increased demand for the respective reaction products (reviewed, e.g., in Tong, B.C. and Barbul, A. (2004) Mini-Rev. Med. Chem. 4, 823-832). Thus, after wounding L-arginine levels become critically low (Albina, J. E. et al. (1988) Am. J. Physiol. 254, E459-E467).

The significant role of arginine metabolism and NO in wound healing lends itself to the possibility of therapeutic modulation of NO. Use of supplemental dietary arginine is proposed as one efficacious way of therapeutic intervention in order to improve wound breaking strength and collagen deposition (reviewed, e.g., in Stechmiller, J.K. et al. (2005) Nutr. Clin. Pract. 20, 52-61; Curran, J.N. et al. (2006), *supra*). However, parenteral administration of L-arginine is significantly hampered by the fact that the small intestinal mucosa is a major source of arginase activity in the body, resulting in the degradation of a substantial portion of dietary arginine during intestinal transit. The remainder enters the portal vein for use in the body. Accordingly, only a limited amount of arginine will finally be available at the wound site in order the improve wound repair.

Furthermore, a beneficial effect of arginine supplementation has only been demonstrated in surgical incision wounds (i.e. artificial wounds), primarily in rodent models. No studies are available so far concerning the effect of arginine supplementation on healing of delayed and chronic wounds. However, in chronic wounds a persistent inflammatory stimulus such as repeated tissue trauma or ischemia causes skin breakdown and prolongs the inflammatory phase of the healing process. The affected area becomes a good medium for bacterial growth. In inflammatory response is initiated by the influx of neutrophils and macrophages into the wound. The process, therefore, bypasses the release of growth factors that signal the healing process to begin as seen in acute injury. A vicious cycle occurs as inflammatory cells secrete cytokines which, in turn, attract more inflammatory mediators. Further tissue damage ensues as the inflammatory phase is delayed.

Thus, there still remains a strong need for improved pharmaceutical compositions for the treatment of wounds which overcome the above limitations. In particular, there is a need for a composition which is both effective in enhancing wound healing, even of chronic wounds, and concomitantly capable of preventing infectious maladies that, in many cases, significantly interfere with the healing process. Furthermore, the pharmaceutical compositions should allow the direct and easy application of the active ingredients to the affected site (i.e. the wound) and, on the other hand, should also be well tolerated by the patients treated.

Accordingly, it is an object of the present invention to provide such improved pharmaceutical compositions for the treatment of wounds.

These and other objectives of the present invention that will become apparent from the description are accomplished by the pharmaceutical composition according to the independent claim. Preferred embodiments are defined by the dependent claims.

### SUMMARY OF THE INVENTION

In a first aspect, the present invention relates to a pharmaceutical composition for topical application, comprising as active ingredients: (a) at least one antiseptic agent and/or at least one agent promoting wound healing; and (b) L-arginine.

In preferred embodiments of the invention, the pharmaceutical composition is a hydrosome composition or a liposome composition.

In further preferred embodiments, the final concentration of L-arginine in the pharmaceutical composition is in the range of 1 mM to 1.000 mM, more preferably in the range of 3 mM and 800 mM, and most preferably in the range of 5 mM and 600 mM.

In other specific embodiments, the antiseptic agent is selected from the group consisting of metal compounds, phenol derivatives, detergents, iodine, and iodine complexes. Preferably, the antiseptic agent is povidone iodine.

In further specific embodiments, the wound healing promoting agent is selected from the group consisting of allantoin, an azulene compound, and a compound from the vitamin B group.

In case, the pharmaceutical composition is a liposome composition, the liposomes may have a substantially uniform size in the range between 20 nm and 20.000 nm, preferably in the range between 50 nm and 4.000 nm, and most preferably in the range between 500 nm and 2.500 nm.

In preferred embodiments, the pharmaceutical composition releases the active ingredients over an extended time period. Particularly preferably, the pharmaceutical composition releases the active ingredients at substantially the same release rate over the release time period.

In other specific embodiments, the pharmaceutical composition further comprises at least one anaesthetic agent.

In some embodiments, the pharmaceutical composition is provided in a dosage form selected from the group consisting of solutions and dispersions.

In alternative embodiments, the pharmaceutical composition is provided in a dosage form selected from the group consisting of oil-in-water emulsions and water-in-oil emulsions. Preferably, the emulsions are selected from the group consisting of ointments, creams, lotions, and balms.

In further preferred embodiments, the pharmaceutical composition is provided in the dosage form of a pharmaceutical gel, more preferably a hydrogel, and most preferably a non-alcoholic hydrogel.

In a second aspect, the present invention relates to the use of a pharmaceutical composition. as defined herein, for the manufacture of a medicament for the treatment of wounds.

### DETAILED DESCRIPTION OF THE INVENTION

The present invention is based on the unexpected finding that the pharmaceutical composition of the present invention comprising as active ingredients a combination of L-arginine and at least one antiseptic agent and/or at least one (further) agent promoting wound healing is ideally suited for the treatment of wound, particularly for the treatment of chronic wounds. In comparison to preparations known from the prior art, the inventive composition enables not only a faster, more efficient and scar-free healing process of wounds but also reliably prevents infections at the site of cutaneous lesion. The particular formulation further allows for a direct (i.e. topical) application of the composition to the affected site. Finally, the inventive composition is easy to administer and also well tolerated by the patients treated.

In the following, the present invention will be described with respect to particular embodiments and with reference to certain drawings but the invention is not limited thereto but only by the claims.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

In a first aspect, the present invention relates to a pharmaceutical composition for topical application, comprising as active ingredients:
(a) at least one antiseptic agent and/or at least one agent promoting wound healing; and
(b) L-arginine.

Accordingly, the pharmaceutical composition of the present invention may comprise the following combinations of active ingredients: L-arginine in combination with one or more antiseptic agents, L-arginine in combination with one or more agents promoting wound healing, and L-arginine in combination with one or more antiseptic agents and one or more agents promoting wound healing.

According to the present invention, it is to be understood that the above-referenced active ingredients are present in the pharmaceutical composition in any amount being effective to achieve the desired pharmacological effect such as to promote wound healing and/or to prevent infectious maladies when administered to a patient. Effective amounts are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention.

Typically, the amount of each active ingredient in the inventive pharmaceutical composition is in the range of 0.1 g to 10 g in 100 g of the composition (i.e. 0.1 % to 10% by weight based on the total weight (w/w) of the composition). This amount of active ingredient corresponds to a concentration of 1 mg to 100 mg per g of the composition.

Arginine (2-amino-5-(diaminomethylideneamino)pentanoic acid) consists of a four-carbon aliphatic straight chain, the distal end of which is capped by a complex guanidinium group. With a p*K*ₐ of 12.48, the guanidinium group is positively charged in neutral, acidic and even most basic environments, and thus imparts basic chemical properties to arginine. Typically, arginine is present (and effective) in the pharmaceutical composition of the present invention in the naturally occurring predominant L-isomer. However, in some embodiments of the present invention, the pharmaceutical composition may also comprise the D-isomer or a mixture, preferably a racemic mixture, of the L- and D-isomers.

According to the invention, the final concentration of L-arginine in the pharmaceutical composition is in the range of 1 mM to 1.000 mM, preferably in the range of 3 mM and 800 mM, and most preferably in the range of 5 mM and 600 mM.

The term "antiseptic agent", as used herein, refers to any antimicrobial substances that are applied to the tissue/skin to reduce the possibility of infectious maladies. These infectious maladies may be of bacterial, viral or fungal (including yeasts) origin. The term "antiseptic agent" does not only include compounds inhibiting the growth of the infective entities (i.e. bacteriostatic, virostatic or fungistatic agents) but also compounds killing said infective entities (i.e. bactericidal, viricidal or fungicidal agents).

A plurality of different antiseptic agents is known for the topical treatment of infectious skin maladies. One group of such agents comprises "classical antibiotics" such as aminoglycosides (e.g., streptomycin, gentamicin), tetracyclins or macrolides (e.g., erythromycin). However, the use of antibiotics may impede granulation and epithelisation of the skin, thereby substantially inhibiting the wound healing process. Furthermore, the infective entities may show primary resistances or acquire secondary resistances against such antibiotics (reviewed, e.g., in Kramer, W. et al. (1999) Indikationen und Auswahlkriterien für lokale Wundantiinfektiva und Wundauflagen im Rahmen der chirurgischen Wundbehandlung. in: Beck, E.G., Eikmann, T., Tilkes, F. (eds) Hygiene in Krankenhaus und Praxis, ecomed, p. 1-16).

Further antiseptic agents to be used in the present invention include *inter alia* alcohols (e.g., ethanol, 1-propanol, and 2-propanol or mixtures thereof, optionally applied along with iodine or cationic surfactants such as benzalkonium chloride or chlorhexidine), phenol derivatives such as alkyl and aryl phenolic compounds (e.g., TCP, trichlorophenylmethyliodosalicyl), organic disinfectants such as formaldehyde-releasing compounds detergents, chinolines, acridines, guanidines, hexahydropyrimidines, quarternary amminoum salts, metal compounds such as mercurochrome, antimicrobial peptides such as the β-defensins, iodine, and iodine complexes.

In specific preferred embodiments, the at least one antiseptic agent is selected from the group consisting of metal compounds, phenol derivatives, detergents, iodine, and iodine complexes.

A particularly preferred antiseptic agent of the present invention is povidone iodine (also known as polyvidone-iodone or PVP-iodone), e.g., a poly(1-vinyl-2-pyrrolidin-2-on)-iodine complex. The use PVP-iodine has the general advantage that the development of resistances against this compound is avoided while retaining at the same time a high anti-microbial efficacy. Moreover, PVP-iodine rarely shows allergenic properties as compared to antibiotics.

The pharmaceutical composition of the present invention may comprise one or more antiseptic agents that may belong to the same class of agents (e.g., two phenol derivatives) or to different classes (e.g., one phenol derivative and one iodine complex).

The term "agent promoting wound healing", as used herein, refers to any compound exerting a beneficial effect in the course of wound healing. Since L-arginine has been shown to have an advantageous effect on the healing of wounds by promoting nitrogen balance it is understood that L-arginine itself is not covered by this term. Agents promoting wound healing comprise *inter alia* substances that are known for promoting granulation, collagen deposition, and re-ephitelization of wounds. These include, for example, growth factors (e.g., platelet-derived growth factor, epidermal growth factor, insulin-like growth factor 1, transforming growth factor α/β), collagen, matrix metalloproteinases, alantoin (5-ureidohydantoin), azulene compounds (e.g., vetivazulene, guaiazulene), and vitamins, particularly from the vitamin B group (e.g., dexpanthenol).

In preferred embodiments, the wound healing promoting agent is selected from the group consisting of allantoin, an azulene compound, and a compound from the vitamin B group.

The pharmaceutical composition of the present invention may comprise one or more wound healing promoting agents which may belong to the same class of agents (e.g., two azulene compounds) or to different classes (e.g., one azulene compound and dexpanthenol).

The pharmaceutical composition according to the present invention may be any therapeutically effective pharmaceutical dosage form for topical administration. Examples of such pharmaceutical dosage forms include *inter alia* solutions, suspensions, dispersions, tinctures, gels, topical sprays, topical foams, gels, water-in-oil emulsions such as ointments, and oil-in water emulsions such as creams, lotions, and balms (cf. below).

The pharmaceutical composition of the present invention may be administered as a single dose or in multiple doses such as two or three individual doses administered during the day, e.g. in the morning, at noon, and at night.

The pharmaceutical composition is typically applied to the skin in a dose rate of 0.01 to 10 mg (of each) active ingredien/cm² skin area, preferably in a dose rate of 0.05 to 5 mg/cm² skin area, and particularly preferably in a dose rate of 0.2 to 1 mg/cm² skin area.

Within the scope of the present invention, it may be possible to apply the pharmaceutical composition to the skin by means of an applicator or dispenser device to ensure administration of a particular amount of the active ingredients comprised to a given skin area (that is, to avoid overdosing of the dosage form). Such (optionally disposable) applicator or dispenser devices are known in the art and include *inter alia* spatulas, pads, sponges, pressurized dispensers, and the like.

In preferred embodiments, the pharmaceutical composition is provided in form of a liposome composition or a hydrosome composition. Within the scope of the invention, the term "liposome" refers to a (spherical) vesicle with an aqueous (hydrophilic) core enclosed by one or more bilayers of lipids, typically of amphiphilic phospholipids. Such bilayer is composed of two opposing layers of lipid molecules arranged in such a way that their hydrophobic hydrocarbon tails face one another, while their charged hydrophilic head regions face the aqueous solutions on either side of the membrane (reviewed, e.g., in Torchilin, V., and Weissig, V. (eds.) (2003) Liposomes - a practical approach, 2nd ed., Oxford University Press, New York, NY).

In contrast, spheres not comprising an aqueous phase in their interior that is surrounded by a monolayer of phospholipids or other amphiphilic compounds are called micelles. In some embodiments, micelles may be present in the pharmaceutical composition according to the invention.

The term "hydrosome", as used herein, refers to aqueous droplets that are immersed in an inert, immiscible, non-aqueous medium and that may be used, for example, for the preparation of hydrogels (Vogt, P.M. et al. (2006) Bums 32, 698-705).

The use of liposomes or hydrosomes as carriers for active ingredients is well established in the art. It is generally assumed that the use of such vehicles allows for deeper penetration of into the skin lesions as compared to conventional preparations (e.g., solutions or tinctures). In this way, the active ingredients are transported in more efficiently way to the damaged area(s). However, it is then surprising that such a radically effective class of compounds as antiseptic agents does not impinge on the healing process of the particularly sensitive damaged tissue and can even suppress the formation of scar tissue, neoplasms, intergrowth, and the like. This beneficial feature may be due to the granulation and epithel-forming effect of the liposomal/hydrosomal preparations.

The term "lipid", as used herein, refers to any hydrocarbon-containing organic compounds to be used in the pharmaceutical composition of the present invention that they are soluble in nonpolar solvents and are relatively insoluble in water. Such compounds include any naturally occurring or synthetically produced fatty acids (i.e. saturated or unsaturated aliphatic monocarboxylic acids having the general formula CH₃(CH₂)ₙCOOH) as well as glycerides (i.e. lipids possessing a glycerol core structure with one or more fatty acyl groups, which are fatty acid-derived chains attached to the glycerol backbone by ester linkages).

Examples of saturated fatty acids include *inter alia* butyric acid, caprylic acid, palmitic acid, and stearic acid. Examples of unsaturated acids include *inter alia* oleic acid and linoleic acid. The term "glycerides" includes mono-, di-, and triglycerides. Examples of such glycerides are *inter alia* phospholids (e.g., phosphatidyl choline, phosphatidyl serine, and diphosphatidyl glycerol), sphingolipids (e.g., ceramide, and sphongomyelin) and sterols such as cholesterol. The lipids may be present in free form or included in a composite such as animal fats (e.g., cod-liver oil), vegetable fats and oils (e.g., cocoa butter, shea butter, olive oil, safflower oil, peanut oil, sesame oil, and sweet almond oil), waxes (such as beeswax, paraffin wax, and wool wax), vaseline® (petroleum jelly, petrolatum) or the like.

Any amphiphilic substances (i.e. phospholipids, cholesterol or glycolipids) generally known in prior art to form liposome membranes can be employed in the context of the invention as long as they are pharmaceutically acceptable for the intended application. Liposome-forming systems comprising phosphatidylcholines (lecithins) are preferred. Such systems may comprise, for example, hydrogenated soy bean lecithin, cholesterol and disodium succinatehexahydrate. Usually one will make sure that the liposome-forming materials do not show any reactivity with the active ingredients comprised in the pharmaceutical composition. Due to its double-bond reactivity high cholesterol contents are usually avoided. In particularly preferred embodiments, hydrogenated soy bean lecithin is employed as the sole lipid membrane-forming agent. Commercially available products such as Phospholipon® 90H (Aventis, Frankfurt, Germany) are also preferred.

Phospholipid-based liposomes may also be used for production of liposomes that discharge their cargo into the skin. Other components that may be used for the formation of liposomes are also known to the person skilled in the art and may be used for the production of compositions according to the invention.

Any established standard methods for forming liposome structures can be used in the context of the invention (reviewed, e.g., in Lauer, A.C. et al. (1996) Adv. Drug Delivery Rev. 18, 311-324). In brief, these methods comprise mechanical agitation of a suitable mixture containing the membrane-forming substance and water or an aqueous solution. Subsequent filtration through suitable membranes is preferred in order to prepare a liposome composition having a substantially uniform size.

The average size of the liposomes according to this invention can vary over a broad range from 1 nm to 100.000 nm. Generally, the size of liposomes should be selected such that a good penetration into the skin is guaranteed. Typically, the liposomes may have a substantially uniform size in the range between 20 nm and 20.000 nm, preferably in the range between 50 nm and 4.000 nm, and most preferably in the range between 500 nm and 2.500 nm.

One exemplary method for producing liposomes to be used in the present invention can be described as follows:
The lipid membrane forming components, e.g. lecithin, are dissolved in a suitable solvent such as chloroform or a 2:1 mixture of methanol and chloroform and are filtered under sterile conditions. Then, a lipid film is produced on a sterile high surface substrate, such as glass beads, by controlled evaporation of the solvent. In some cases, it can be quite sufficient to form the film on the inner surface of the vessel used in evaporating the solvent, without using a specific substrate to increase the surface.

An aqueous system is prepared from electrolyte components and the combination of active ingredients to be incorporated in the liposome preparation. Such an aqueous system may comprise, for example, 10 mM sodium hydrogen phosphate and 0.9% (w/v) sodium chloride, at ph 7.4; the aqueous system will further comprise at least the desired amount of the active ingredients. Often, the aqueous system will comprise an excess amount of said active agents.

The liposomes are formed by mechanically agitating the aqueous system and the lipid film. At this stage, further additives such as sodium cholate can be added to improve liposome formation. Liposome formation can also be influenced by mechanical action such as pressure filtration through e.g. polycarbonate membranes, or centrifuging. Generally, the raw liposome dispersion will be washed, e.g. with electrolyte solution as used in preparing the above-described solution of the active ingredients.

If liposomes having the required size distribution have been obtained and washed, they can be re-dispersed in an electrolyte solution as already described. The dispersion may be freeze-dried and may be lyophilyzed. It can, prior to use, be reconstituted by addition of water and suitable mechanical agitation at the transition temperature of the lipid component (that is, for example, 55°C for hydrogenated soy bean lecithin).

Within the scope of the present invention, the combination of active ingredients comprised in the pharmaceutical composition may either be completely encapsulated inside the liposome or hydrosome particles or completely attached to the outer surface of the liposome or hydrosome particles or partly encapsulated inside and partly attached to the outer surface of the liposome or hydrosome particles. The skilled person is aware of various methods for "loading" the liposome or hydrosome particles with the active ingredients (see, for example, European patents EP 0 639 373 B1 and EP 1 079 806 B1).

In preferred embodiments, the pharmaceutical composition releases the active ingredients over an extended time period, typically a time period between 1 hour and 24 hours. Particularly preferably, the pharmaceutical composition releases the active ingredients at substantially the same release rate over the release time period.

A range of matrix forming natural and synthetic polymers is available to prolong or modify the release of active ingredients such as xanthan gum, galactomannan polymers, alginate, cellulose derivatives (methycellulose, hydroxypropyl cellulose and hydroxypropyl methylcellulose etc.), acrylic and methacrylic co-polymers and combinations thereof. This range of polymers enables formulators to obtain the desired release profile.

Alternatively, the pharmaceutical composition may contain one or more excipients (that is, carriers, solvents or other adjuvants), which are suitable for regulating or modifying the release of the active ingredients. Suitable excipients for include hydrophobic release controlling agents and/or hydrophilic polymers.

The hydrophobic release controlling agents may preferably be selected from the group comprising ammonium methacrylate copolymers, methacrylic acid copolymer, polyacrylate, polyvinyl acetate, ethylcellulose, cellulose acetate, cellulose propionate, cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl actylate), poly(octadecyl acrylate), waxes, fatty alcohols, fatty acid esters, and hydrogenated castor oil.

The inventive pharmaceutical composition may also contain an extended release polymer layer with a hydrophilic polymer being selected from the group consisting of carboxymethyl cellulose, guar gum, hydroxyethyl cellulose, hydroxypropyl cellulose, hydroxypropylmethyl cellulose, methylcellulose, and povidone. Alternatively, the pharmaceutical composition may contain an extended release polymer layer with a hydrophobic material being selected from the group consisting of carnauba wax, ethylcellulose, glyceryl palmitostearate, hydrogenated castor oil, hydrogenated vegetable oil, microcrystalline wax, polymethacrylate, and stearic acid.

Alternative materials for preparing pharmaceutical compositions according to the present invention showing a defined release profile as well as methods for preparing such compositions are well established in the art.

In case, at least part of the active ingredients comprised in the pharmaceutical composition are attached to the outer surface of the liposome or hydrosome particles a marked initial release effect may observed in addition to the slower, protracted release of the active ingredients. Without wishing to be bound to any theoretical explanation, it is assumed that the active ingredients are (loosely) associated with the membrane of the particles or are forming a layer on the liposomal surface, which layer partly or even fully coats the particles externally. The type and amount of this initial effect may be influenced *inter alia* by the choice of the concentration parameters, the components used for forming the particles, and the like.

The pharmaceutical composition according to the present invention may comprise additional classes of active ingredients such as anti-inflammatory agent. In specific embodiments, the composition further comprises at least one anaesthetic agent. Such anaesthetic agents include *inter alia* opioids (e.g., alfentanil, fentanyl, remifentanil, and sufentanil), barbiturates (e.g., methohexital, thiopental), benzodiazepines (e.g., diazepam, lorazepam), etomidate, ketamine, muscle relaxants (e.g., rapacuronium, pancuronium), and local anaesthetics (e.g., lidocaine, dibucaine).

The pharmaceutical composition according to the present invention therapeutically effective pharmaceutical dosage form for topical administration, for example, solutions, suspensions, dispersions, tinctures, gels, topical sprays, topical foams, gels, water-in-oil emulsions such as ointments, and oil-in water emulsions such as creams, lotions, and balms

All these topical pharmaceutical dosage forms as well as methods for their preparation are well established in the art (see, for example, Niedner, R., and Ziegenmeyer, J. (1997) Dermatika. Therapeutischer Einsatz, Pharmakologie und Pharmazie. Wissenschaftliche Verlagsgesellschaft mbH, Stuttgart, Germany; Gennaro, A.L. and Gennaro, A.R. (2000) Remington: The Science and Practice of Pharmacy, 20th Ed., Lippincott Williams & Wilkins, Philadelphia, PA; Niazi, S.K. (2004) Handbook of Pharmaceutical Manufacturing Formulations, CRC Press, Boca Raton, FL).

In some embodiments, the pharmaceutical composition is provided in a dosage form selected from the group consisting of solutions and dispersions.

In alternative embodiments, the pharmaceutical composition is provided in a dosage form selected from the group consisting of oil-in-water (o/w) emulsions and water-in-oil (w/o) emulsions. Preferably, the emulsions are selected from the group consisting of ointments (w/o), creams (o/w), lotions (o/w), and balms (o/w).

The term "emulsion", as used herein, denotes a mixture of two immiscible (i.e. not mixable) substances. One substance (the dispersed phase) is dispersed (i.e. distributed) in the other (the continuous phase) by the presence of one or more emulsifying agents. The term "oil-in-water emulsion", as used herein, refers to formulations composed of small droplets of a lipid phase dispersed in a continuous aqueous phase. *Vice versa*, the term "water-in-oil emulsion", as used herein, refers to formulations composed of small droplets of an aqueous phase dispersed in a continuous lipid phase. In general, oil-in-water are not only more comfortable but also pharmaceutically/cosmetically better acceptable as compared to water-in-oil emulsions as they are less greasy when applied on the skin and more easily washed off when using water. By employing such an oil-in-water emulsion the penetration of amphiphilic compounds such as the tri-substituted glycerol compounds of the invention through the skin is improved as compared to formulations having only an aqueous phase, since the presence of a lipid phase is assumed to aid in crossing the hydrophobic core of biological membranes.

Preferred oil-in-water emulsions of the invention are selected from the group consisting of creams, lotions, and balms. These formulations primarily differ with regard to their respective viscosities. A cream is a semi-solid emulsion, that is, it has a medium viscosity. In contrast, a lotion is a low- to medium-viscosity preparation intended for application to unbroken skin. Finally, a balm (also referred to as liniment) has a similar viscosity as a lotion (i.e. being significantly less viscous than a cream) but unlike a lotion a balm is applied with friction, that is, a liniment is always rubbed in. Preferred water-in-oil emulsions of the invention are ointments which are also characterized by a semi-solid appearance and medium to high viscosity.

Preferably, the emulsions according to the invention comprise one or more emulsifiers in a total amount of 0.5% to 15% (w/w) based on the total weight of the dosage form. Whether an emulsion turns into a water-in-oil emulsion or an oil-in-water emulsion depends on the volume fraction of both phases and on the type of emulsifier. Generally, the Bancroft rule applies: emulsifiers and emulsifying particles tend to promote dispersion of the phase in which they do not dissolve very well. In other words, the phase in which an emulsifier is more soluble constitutes the continuous phase. Thus, for the preparation of oil-in-water emulsions water-soluble emulsifiers are preferred, and *vice versa.*

Preferred emulsifiers to be used in the present invention are selected from the group consisting of sorbitan esters (also referred to as Span®), polyoxyethylene sorbitan esters (also referred to as polysorbates; Tween®), and glyceryl esters. Examples of sorbitan esters include *inter alia* sorbitan monooleate, sorbitan monostearate, sorbitan monolaurate, sobitan trioleate, and sorbitan tristearate. Examples of polyoxyethylene sorbitan esters include polyethylene glycol (PEG) sorbitan esters such as *inter alia* PEG-(5)-sorbitan monooleate, PEG-(4)-sorbitan monostearate, PEG-(4)-sorbitan monolaurate, PEG-sobitan trioleate, and PEG-sorbitan tristearate. Examples of glyceryl esters include *inter alia* glyceryl monostearate, glyceryl monolaurate, and glyceryl tristearate.

Other suitable emulsifiers include *inter alia* phosphatidylglycerols, alkyl alcohols, poloxamers (also referred to as Pluronic®/Synperonic®), poloxamin (also referred to as Tetronic®), sodium laurylsulfate, sodium cetylstearylsulfate, and potassium oleate.

In further preferred embodiments of the invention, the pharmaceutical composition is provided in the dosage form of a pharmaceutical gel, more preferably a hydrogel, and most preferably a non-alcoholic hydrogel (i.e. a hydrogel not comprising alcoholic com pounds).

The term "(pharmaceutical) gel", as used herein, refers to a colloidal system in which a porous network of interconnected nanoparticles spans the volume of a liquid medium. In general, gels are apparently solid, jelly-like materials. Both by weight and volume, gels are mostly liquid in composition and thus exhibit densities similar to liquids, however have the structural coherence of a solid.

The term "hydrogel", as used herein, refers to a gel made of one or more cross-linked water-swellable (hydrophilic) gel-forming polymers such as polysaccharides or polyacrylic acid derivatives. The gel-forming polymers may be naturally occurring polymers, synthetic polymers or mixtures thereof. Hydrogels may be prepared from hydrosome compositions and may comprise more than 99% water. When applied to the skin the water bound in such a hydrogel does not evaporate as fast as from a solution. Due to the thus prolonged contact period the skin becomes moistened which, in turn, results in an improved susceptibility for the uptake of active ingredients present in the hydrogel (i.e. an increased penetration through the skin). This phenomenon is also referred to as "occlusion effect".

Typically, such gel-forming polymers for preparing hydrogels have an average molecular weight of 1000 to 50000 Dalton, preferably of 1000 to 30000 Dalton. A hydrogel of the invention may also be characterized by its rheological properties. Typically, it has an initial shear modulus of 0.005 to 200 kPa, preferably of 0.05 to 100 kPa. The "shear modulus", also referred to as the modulus of rigidity, is defined as the ratio of shear stress to the shear strain and provides a measure for the strength of a given material.

Additionally or alternatively, it may also be possible to characterize a hydrogel by its flow behavior such as by its viscosity coefficient η as determined by the flow models of Bingham, Casson, Herschel-Bulkley and Ostwald, respectively, all of them well known in the art (see, e.g. Gosh, T.K. et al. (1997) Transdermal and topical drug delivery systems. CRC Press, Boca Raton, FL, USA; Fairclough, J.P.A., and Norman A.I. (2003) Annu. Rep. Prog. Chem., Sect. C: Phys. Chem. 99, 243-276).

In another preferred embodiment, the hydrogel comprises one or more gel-forming polymers in a total amount of 0.1% to 15% (w/w) based on the total weight of the hydrogel. Particularly preferably, the one or more gel-forming polymers are selected from the group consisting of cellulose derivatives, polyacrylic acid derivatives, and gums. Examples of cellulose derivatives include *inter alia* methylcellulose, ethylcellulose, hydroxyethyl cellulose, and carboxymethyl cellulose. Examples of polyacrylic acid derivatives include *inter alia* polyacrylic acid, polymethylacrylate, and polyethylacrylate. Examples of gums (also referred to as "rubbers") include *inter alia* agar, alginic acid, glucomannan, arabic gum, sodium alginate, and tragacanth.

The pharmaceutical composition of the present invention may further comprise one or more other customary agents including *inter alia* additives and adjuvants, carrier materials, wetting agents, anti-oxidants, preservatives, buffer substances (for adjusting a pH value of the pharmaceutical composition, e.g., in the range between 7.0 and 7.6, preferably a pH of 7.4), solvents or solubilizers, agents for achieving a depot effect, and viscosity-adjusting additives, all of them well known in the art (cf. the references cited above). The person skilled in the art will select these additional agents in such a way that the ability of the pharmaceutical compositions obtained complies with the intended application. Such customary agents may also comprise salts that allow for the regeneration of an active ingredient, for example the released halogen atom in case of halogen-releasing compounds. In case, PVP-iodine is one of the active ingredients employed such a salt additive may be KIO₃. Other additives that mediate or enhance penetration of the liposomes into the skin may also be part of the inventive pharmaceutical compositions. Such additives comprise, e.g., dimethylsulfoxide (DMSO).

In a second aspect, the present invention relates to the use of a pharmaceutical composition of any of claims 1 to 14 for the manufacture of a medicament for the treatment of wounds.

The invention is further described by the figures and the following examples, which are solely for the purpose of illustrating specific embodiments of this invention, and are not to be construed as limiting the scope of the invention in any way.

### EXAMPLES

### Embodiment Example I

In a 1000 ml glass flask, provided with glass beads for increased surface, 51.9 mg cholesterol and 213 mg hydrogenated soy bean lecithin were dissolved in a sufficient amount of a 2:1 (v/v) mixture of methanol and chloroform. The solvent was then evaporated under vacuum until a film formed on the inner surface of the flask and on the glass beads.
2.4 g PVP iodine (containing about 10% available iodine) were separately dissolved in 12 ml water.

Again in a separate vessel, 8.77 g sodium chloride and 1.78 g Na₂HPO₄·2H₂O were dissolved in 400 ml water. Water was further added up to a total volume of 980 ml, and then approximately 12 ml 1N hydrochloric acid was added to adjust pH to 7.4. The volume of this solution was adjusted with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. The resulting liposome formulation was separated from the hydrated lipids in the flask. The product was centrifuged and the supernatant was discarded. The saccharose solution was added ad 12 ml, and the product was again centrifuged. Afterwards, the supernatant was again discarded. At this stage, a further washing step, using the saccharose solution or the sodium chloride buffer solution could be carried out.

After the last centrifugation step and discarding of the supernatant, 12 ml sodium chloride buffer solution was added, and the liposomes were homogenously dispersed therein. The product was then aliquoted into vials, each vial containing 2 ml liposome dispersion, and the vials were then subjected to a freeze-drying step.

After the freeze-drying, each vial comprised about 40 mg solids.

In the method of Embodiment Example I, due to the high percentage of solids the PVP iodine solution used is rather viscous and thus more difficult to handle.

The use of unsaturated compounds like cholesterol will have to be carefully considered as well, since iodine may react with such unsaturated compounds. For many applications, only small amounts of such compounds will be employed, or liposomes will be used that can be formed in the absence of such compounds (as detailed, e.g., in the following examples).

### Embodiment Example II

In a 2000 ml flask provided with glass beads to increase surface, 173 mg hydrogenated soy bean lecithin and 90 mg disodium succinate were dissolved in approximately 60 ml of a 2:1 (v/v) mixture of methanol and chloroform. The solvent was removed under vacuum until a film was formed.

4 g PVP iodine (10% available iodine) were dissolved in 40 ml of the sodium chloride buffer solution described in Embodiment Example I, and were added to the lipid film in the flask. The flask was then shaken until the film dissolved and liposomes were formed.

The product was centrifuged and the supernatant liquid was discarded.

To the liposome pellet thus obtained, further 40 ml sodium chloride buffer solution was added, and the centrifuging step was repeated. The supernatant was again discarded. At this stage, the washing step could be repeated where necessary.

After the final centrifugation and decantation step, 40 ml sodium chloride buffer solution was again added to the precipitated liposomes. The homogenous dispersion was then aliquoted into vials, each vial containing about 2 ml liposome dispersion, and the vials were then subjected to a freeze-drying step. This resulted in approximately 200 mg freeze-dried solids per vial.

The freeze dried compositions are placed in a polypropylene container for storage.

From the freeze-dried solid compositions of Embodiment Examples I and II, further preparations were made as described below.

The above-described method, like that in Embodiment Example I, uses a hydrating step after film formation in the presence of organic solvents and aims at inclusion rates of 5% to 15%. These methods generally produce rather large and often multi-lamellar liposomes.

The above-described methods can be modified by a high pressure filtering step through a suitable membrane such as a polycarbonate membrane after the raw liposomes have been formed or after any of the subsequent washing steps or directly by using high pressure homogenization. This results in the production of much smaller, unilamellar liposomes at increased amounts of encapsulated agent.

Instead of high pressure homogenization, other prior art methods known to provide small uniform sized liposomes can be employed.

### Embodiment Example III

A hydrophilic oil-in-water (o/w) cream was prepared from 10 g hydrogenated soy bean lecithine/PVP iodine liposomes as described in Embodiment Example II. These liposomes were mixed with 4 g Polysorbate 40 ™, 8 g cetylstearyl alcohol, 8 g glycerol, 24 g white vaseline, and water ad 100 g.

### Embodiment Example IV

An amphiphilic cream was prepared from 10 g hydrogenated soy bean lecithine/povidone iodine liposomes as described in Embodiment Example II. The liposomes were mixed with 7.5 g medium chain length tryglyceride, 7 g polyoxyethyleneglycerol monostearate, 6 g cetylstearyl alcohol, 8 g propylene glycol, 25 g white vaseline, and water ad 100 g.

### Embodiment Example V

A hydrophilic ointment which can be rinsed off with water was prepared using 10 g of liposomal PVP iodine as described in Embodiment Example II. The liposomes were mixed with 55 g Macrogol 400 ™, 25 g Macrogol 4000 ™, and water ad 100 g.

### Embodiment Example VI

A hydrogel was prepared from 4 g liposomal PVP iodine as described in Embodiment Example II. The liposomes were mixed with 0.5 g Carbopol 980 NF ™, sodium hydroxide ad pH 7.0, and water ad 100 g. Further modifications of the above-described embodiments may be made.

For example, the creams of Embodiment Examples IV and V may further comprise an agent known to promote the healing of wounds, such as allantoin.

Such an agent will be added in a pharmaceutically useful concentration, in the case of allantoin in the range of 0.1-0.5 g per 100 g of cream. The wound healing agent can be incorporated in the cream base, in which case it will largely be outside the liposomes. It can, however, be partly or mostly incorporated in the liposomes, in which case it will be added at a corresponding suitable stage of the liposome composition method.

Similar alternatives are easily envisaged on the basis of the further Embodiment Examples.

For application of the preparations according to the present invention to a patient known systems can be used, such as *inter alia* pneumatic pump applicators, two-chamber gas pressure packs, and aerosol spray dispensers.

In a pneumatic pump applicator, a bellows device is provided between an upstream and a downstream valve, both valves operating one way in the same direction. A supply of pharmaceutical preparation, such as an ointment or gel, is contained in a reservoir upstream of the valves-and-bellows device.

When compressing the bellows, the downstream valve opens and permits a dosed amount of preparation to leave the device for application. When the bellows is extended, this valve shuts and prevents reentry of the preparation. At the same time, the upstream valve opens and permits preparation from the reservoir to enter into the bellows, for release through the downstream valve upon the next compression step of the bellows.

The reservoir is sealed by a closure element which can move through the reservoir like a piston moves in a cylinder. By the stepwise emptying of the reservoir, this closure element is sucked into the reservoir, so that the remaining amount of pharmaceutical preparation in the reservoir is always sealed off, while at the same time the reservoir can be emptied. Such a device is useful for pasty preparations, creams, ointments and the like.

In a two-chamber gas pressure pack, the pharmaceutical preparation is contained in a bag of flexible plastics film material. Often, this material is high pressure polyethylene.

The bag is contained inside a gas tight pressure vessel which further contains a supply of pressurizing gas, very often a compressed inert gas like nitrogen or air.

The plastic film bag has only one outlet which is in sealed (i.e. gas-tight) connection to the interior wall of the pressure vessel, surrounding a single opening thereof. The pressurized gas in the vessel tends to compress the bag, driving the pharmaceutical preparation inside the bag out through the opening of the bag and thus through the opening of the vessel. A valve and optionally a spray-head device are provided in the vessel mouth. Operating the valve releases a spray mist, a jet of liquid or a portion of flowable solid such as cream. By using such a system solutions, emulsions, creams, ointments and gels can be dosed and applied.

Preferably, the applicators are designed to contain a separate chamber for the dry composition and a chamber for the liquid medium, which can be combined to obtain one chamber containing the dry composition and the liquid medium which via shaking give the applicable preparation. Preferably one of the chambers contains additionally beads of ceramic, metal or plastic materials to enhance the blending via shaking.

### Embodiment Example VII

A dry liposomal composition was prepared. The amounts shown in Table I were used either for analytical or scale up compositions.

**TABLE I**

| **Pos.** | **Substance** | **Amount (g/100g)** | **Scale up (kg/1500kg)** |
|---|---|---|---|
| A | H₂O | 15.0 | 200.0 |
| A | Phospolipon 90 H | 3.0 | 45.0 |
| B | H₂O | 40.0 | 600.0 |
| B | Carbopol® 980 NF | 1.5 | 22.5 |
| C | H₂O | 2.0 | 30.0 |
| C | KIO₃ | 0.0708 | 1.09 |
| D | H₂O | 20.0 | 300.0 |
| D | PVP-iodine 30/06 Available iodine (10%) | 3.0 | 45.0 |
| E | H₂O | 2.5 | 50.0 |
| F | H₂O | 2.5 | 50.0 |
| G | H₂O | 4.6 | 69.0 |
| G | NaOH solid | 0.46 | 6.9 |
| I | Citric acid, H₂O free | 0.1065 | 1.059 |
| I | Na₂(HPO)₄, H₂O free | 0.225 | 3.37 |
| I | H₂O | 3.0 | 45.0 |
| H | H₂O | ad 100 | ad 1500 |

| | | | |
|---|---|---|---|
| "Pos." denotes the position (see also Table II below). Phospholipon® 90 H was purchased from Aventis (Germany). Carbopol® 980 NF was purchased from Noveon Inc. (USA) or Gattefossé (Germany), and PVP Iodine 30/06 was purchased from BASF (Germany). | | | |

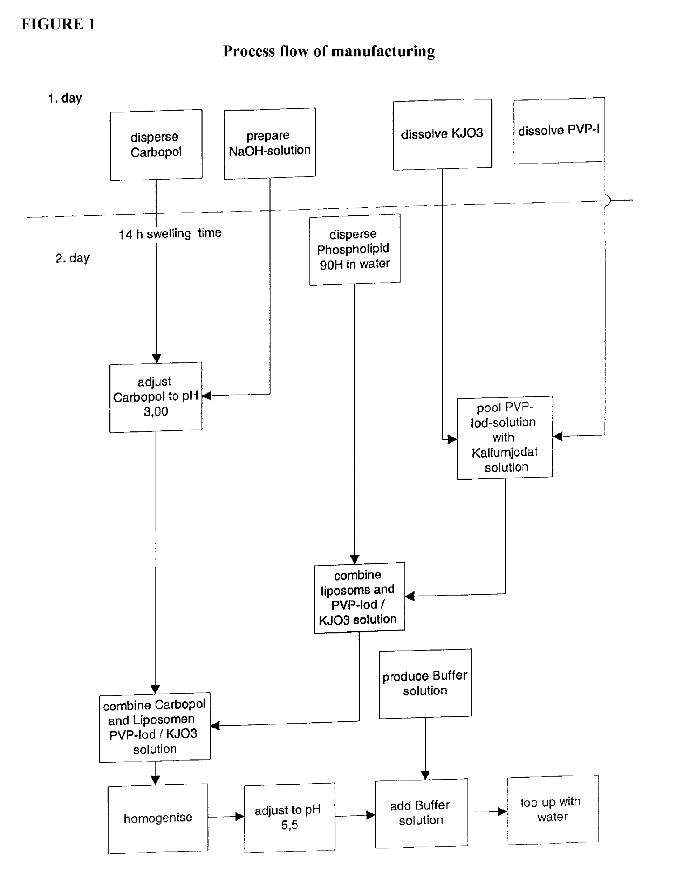

In Table II, middle column, the exact order of steps and the parameters of each step are given (see also Figure 1). In the right column non-exclusive alternatives are discussed. All steps were performed at room temperature except when indicated otherwise. All substances were of a purity grade common for pharmaceutical preparations such as described, e.g., in the British Pharmacopeia.

**TABLE II**

| **No.** | **Embodiment example VII** | **Alternatives** |
|---|---|---|
| 1 | Carbopol 980 NF is mixed into H₂O without agglomeration (Pos. B). Stirring for 30 min at approx. 30 upm (units per minute) in conventional stirrer. Visual control for polyacrylic acid-agglomerates. | Substances: other gel-forming substances may be used. |
| | | Homogenization time can vary: shorten to 1min prolong to 10 min (caution: gel structure may be destroyed) |
| | If necessary, homogenize gel in conventional homogenizer for 2 min at 3000 upm. | Stirring time can be altered as desired. Only condition is that gel is free of agglomerates |
| | Subsequently stir gel for 30 min at 30 upm in conventional stirrer. Eventually control again for polayacrylic acid-agglomerates. | at the end. |
| | If present, remove them and stir again for 15 min at 30 upm. Eventually homogenize again. | Swelling time may be altered from 15 min to 5 days. Preferably, the gel has formed before other substances are added. Adjustment of pH to 2-8 may be performed |
| | Let gel swell for at least 14 h. | at this stage. Adjustment to pH 3-6 is preferred. |
| 2 | Dissolve H₂O and KIO₃ completely in a suitable vessel (Pos. C). Alternatively, a 30-40% KIO₃ solution may be used. | H₂O-temperature may be adjusted to anywhere between ambient temperature and 100°C. |
| | | KIO₃ is not obligatory. |
| 3 | Dissolve NaOH completely in H₂O (Pos. G). | NaOH is used in concentrations common for pharmaceutical preparations. |
| | | Other bases or substances may also be used for formation of the gel structure as, e.g., KOH, triethanolamine, 2-amino-2- methyl-1-propanol, tris(hydroacnemethyl)-aminoethan, 2-hydroacnepropyl-ethylen-diamine, and di-isopropanolamine. |
| 4 | Mix PVP-iodine into H₂O while stirring at 1000 upm in conventional stirrer (Pos. D). | Stirring time and speed can be altered arbitrarily. |
| | Stir mixture for further 60-70 min at 1000 upm until it is completely dissolved. | Important: PVP-lodine has to be dissolved completely. |
| 5 | Warm H₂O to 65°C while stirring with 1000 upm in conventional stirrer. Then, add slowly Phospholipon® 90 H (Pos. A). Take care that no agglomerates are formed. | Possible temperature range: 40 °C - 120 °C. 50 °C - 75 °C is preferred because of phase transition temperature. |
| | Stir dispersion for further 90 min at 65°C to 70°C and 1000 upm. | Other liposome-forming materials or mixtures thereof may be used. |
| | | Stirring time and speed is dependent on |
| | Subsequently cool liposomal dispersion to < 30°C while stirring at 500 upm | equipment. A complete dispersion has to be achieved. Apparatus of the rotor/stator principle, high pressure homogenizers, ultrasound or extrusion technology may also be used for stirring. |
| 6 | By adding the NaOH-solution (No. 3) the gel is adjusted to a pH of 3.0 (± 0.2). | Further processing to a gel may be feasible without pH pre-adjustment and is dependent on the gel-forming substance |
| 7 | The KIO₃ solution (No. 2) is added to the PVP-lodine solution (No. 4) while stirring at 1000 upm. Stirring continued for at least 60 min. | Reaction between KIO₃ and PVP-iodine is time dependent. To ensure a complete reaction, the stirring time has to be adapted accordingly. Thus, stirring time may be between 10 min and 2 h |
| 8 | The PVP-iodine/KIO₃ solution is pumped into the liposomal dispersion (No. 5). Subsequently it is stirred for 30 min at 1000 upm. | Stirring time is variable depending on until when a homogeneous mixture has formed. |
| 9 | The PVP-iodine- KIO₃-liposomes-dispesion is added to the gel (No. 6). It is stirred for 30 min at 30 upm. Subsequently homogenization is performed by forced circulation pumping for 2 min at 2800 upm. After checking for agglomerates, it may be homogenized for further 1-2 min. | Stirring time is variable depending on until when a homogeneous mixture has formed. Stirring time should be as short as possible so that gel structure gets not disrupted. |
| 10 | Remove agglomerates if present. Add 50.0 kg NaOH-solution (in the scale up, point 3) while stirring at 30 upm. Stir for further 30 min at 30 upm at <30-C. Cool if necessary. | Adjust stirring time and speed to gel quality. Amounts of NaOH may vary. Adding of base by step wise adjustment until desired pH is achieved. |
| | Determine pH and add additional NaOH until a pH of 5.5 (±0.2) is achieved. After each adding step stir for 20 min. After each adding step homogenize by circulation pressure pumping for 15 s at 1000 upm. After adjustment of pH, stir for further 15 min at 30 upm. Check pH and correct if necessary. After successful pH adjustment, add remaining H₂O amount which depends on the amount of NaOH used. | |
| 11 | Mix buffer solution at 30°C while stirring until it is completely dissolved (Pos. I). | Temperature can be raised to 40°C. Other suitable buffers may also be used. |
| 12 | Buffer solution is added to the product (No. 10) while stirring for 15 min at 30 upm. Degas by application of vacuum. | The desired product quality (storage stability) is achieved by addition of the buffer. Stirring time is variable depending on until when a homogeneous mixture has formed. Degasing may be achieved by other means than vacuum. |
| 13 | Add the remaining H₂O-amount (Pos. H) and stir for 30 min at 25 upm Optionally homogenization may be performed by circulation pressure pumping for 15 s at 1000 upm. Stir for another 30 min. Check visually for agglomerates. | Stirring time is variable depending on until when a homogeneous mixture has formed. |

Positions E and F of Table I are used for washing the respective KIO₃ and the PVP-iodine vessels (points 2 and 4 of Table II).

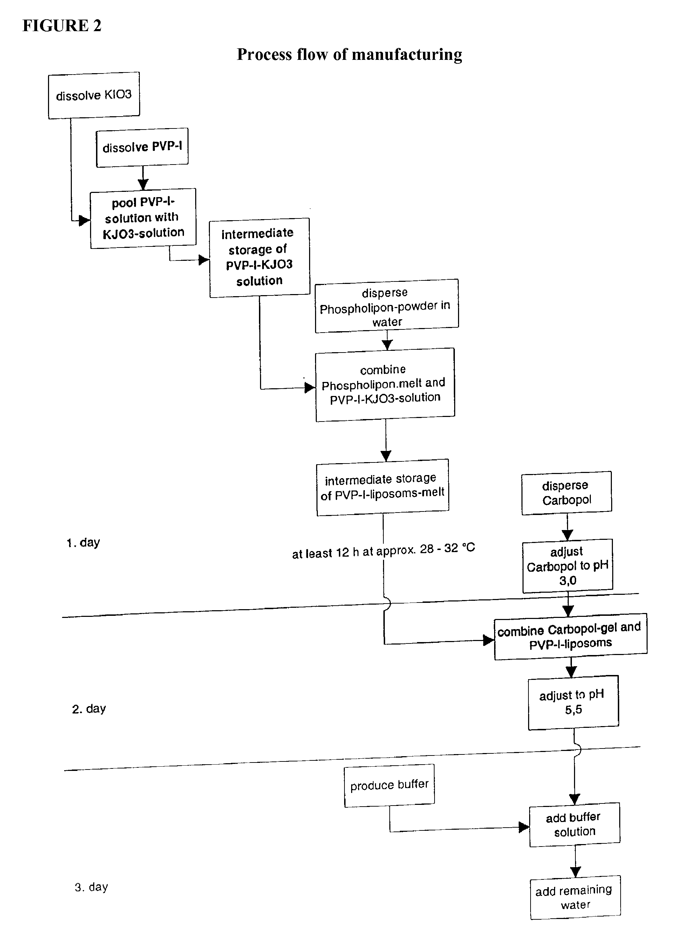

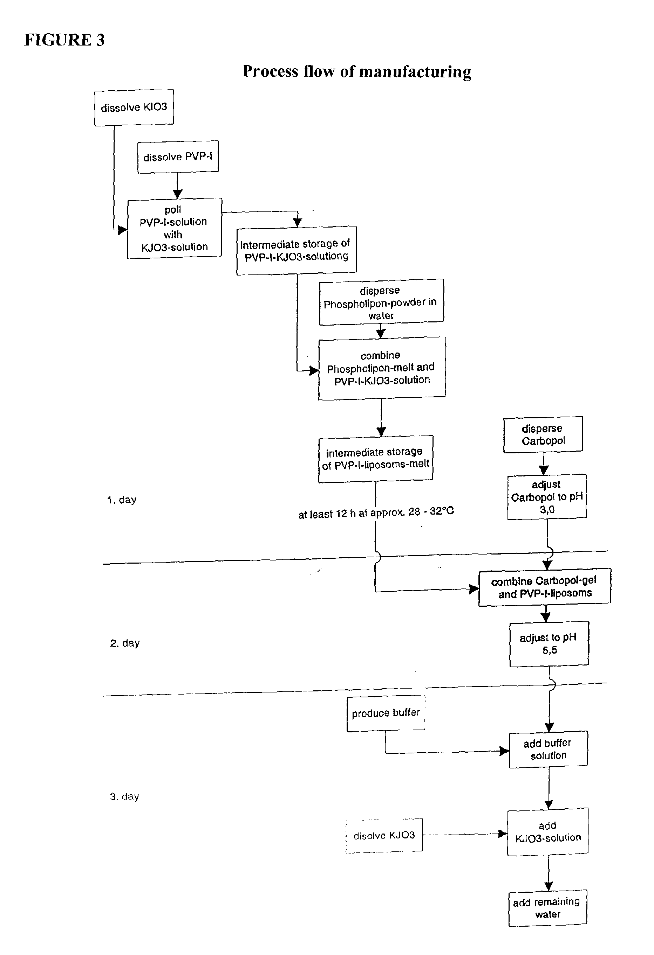

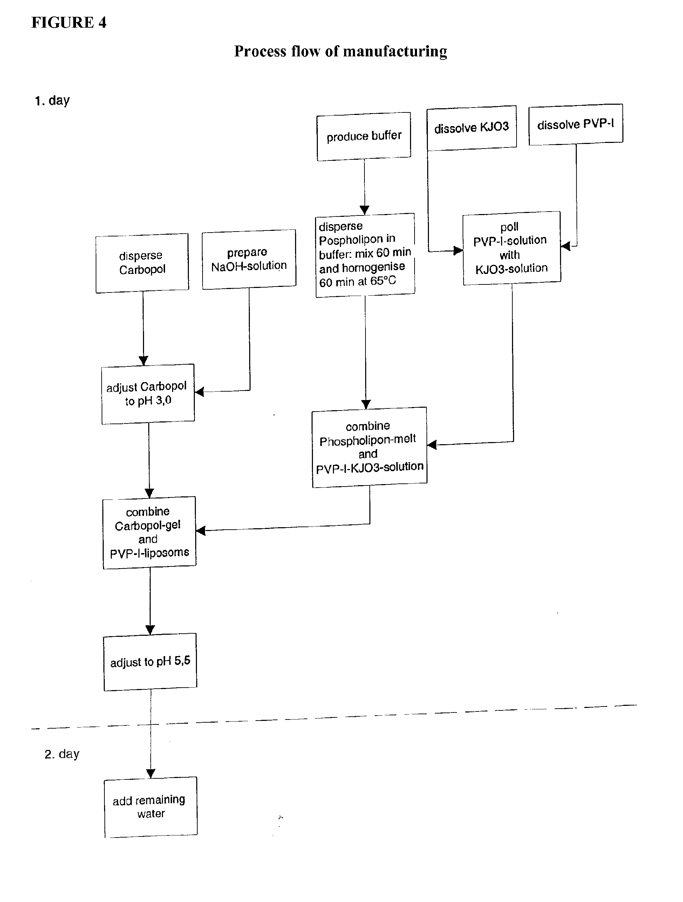

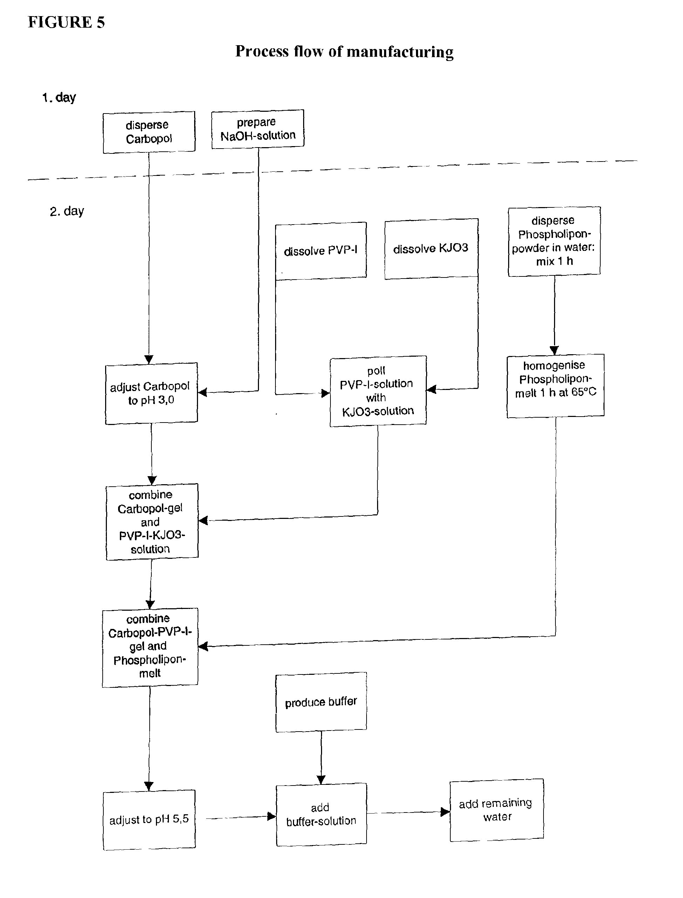

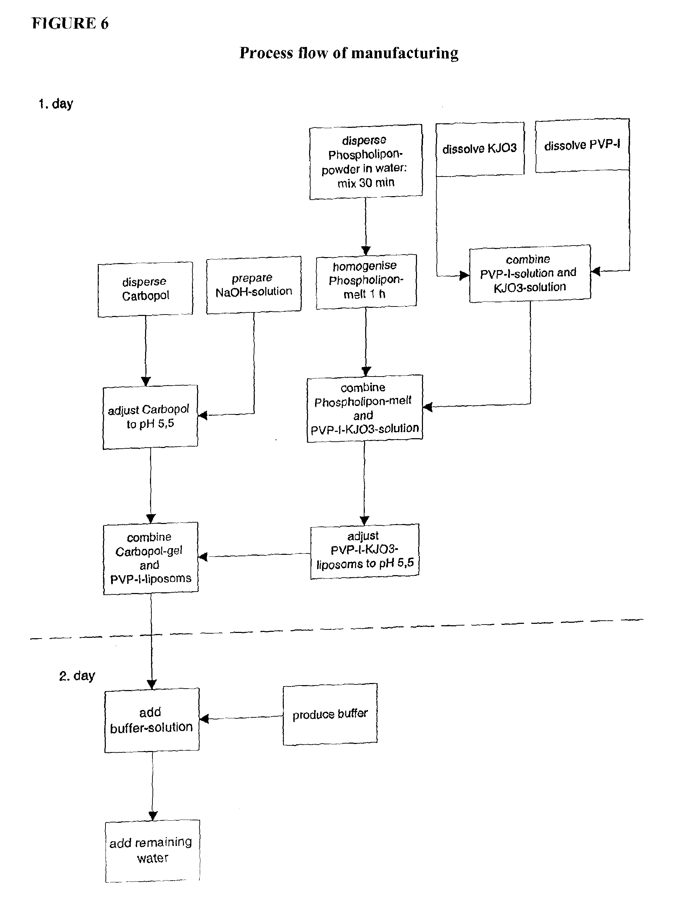

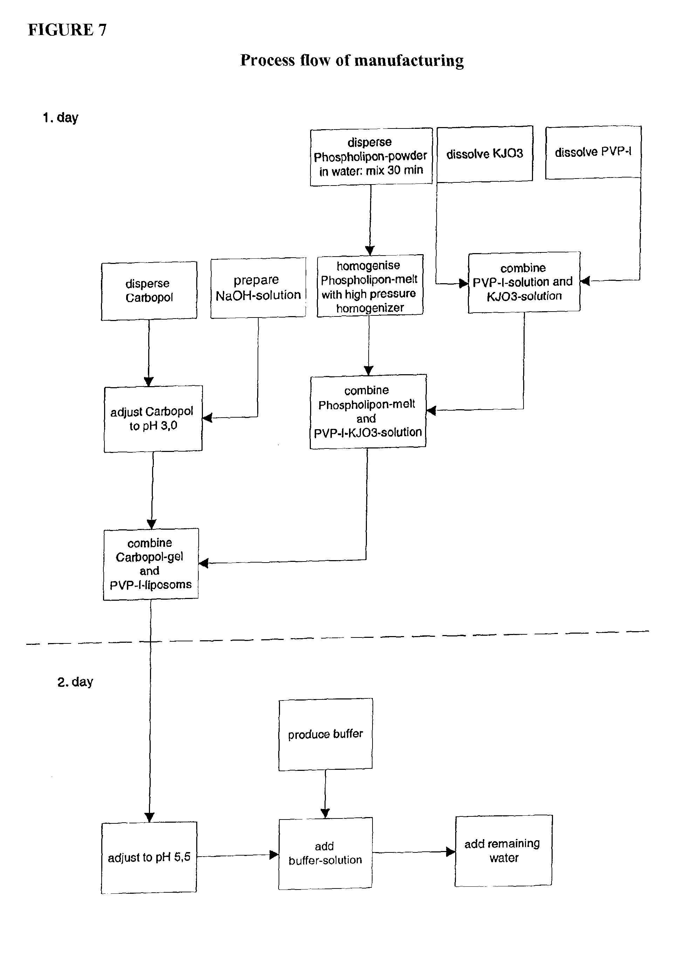

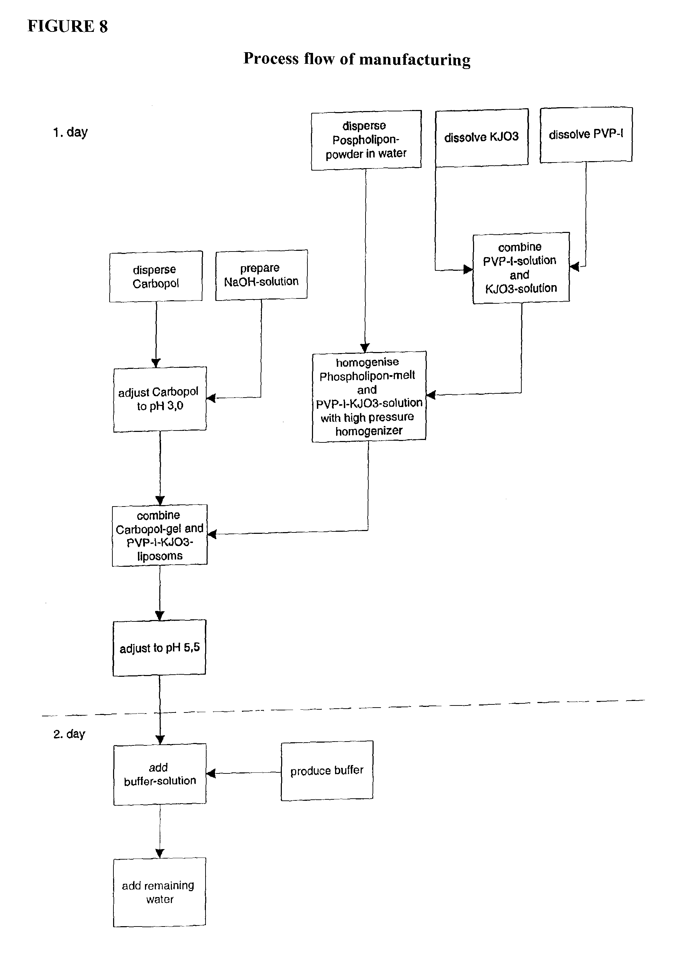

As mentioned above, the hydrogel formulation is produced according to the method set out in Table 2 and Figure 1. Alternative methods become obvious from the flow charts of Figures 2 to 8. The individual steps may be performed as set out above.

Subsequently, the following efficiency tests were carried out using the inventive preparations:

### Test I

This was an *in vitro* test of the bactericidal effect conferred by a povidone iodine liposome preparation according to the invention. The test is based on the quantitative suspension test as described in "Richtlinien der Deutschen Gesellschaft für Hygiene und Mikrobiologie" (1989). In this test, the bactericidal agent is used to kill *Staphylococcus aureus* (American Type Culture Collection number ATCC 29213) - a bacterial species causing major problems in hospital hygiene.

The liposome preparation used was that of Embodiment Example I. At different contact times between 1 and 120 min, the minimum concentration of the preparation in water that was capable of killing the staphylococci was determined.

The results are shown in Table 3 below.

The results demonstrate that at short contact times (1- 4 min) the bactericidal concentration is less than 0.060%, whereas that at long contact times (120 min) it is as low as 0.007%.

**TABLE III**

| **Contact time (min)** | **Bactericidal concentration** |
|---|---|
| 1,2,3,4 | ≥ 0.060% |
| 5,30,60 | ≥ 0.015% |
| 120 | ≥ 0.007% |

### Test II

An anti-viral and anti-chlamydial activity of liposomal PVP-iodine has been described in cell cultures by Wutzler, P. et al. (2000) Ophthalmic Res. 32, 118-125. In cell cultures, liposomal PVP-iodine is highly effective against herpes simplex virus type 1 and adenovirus type 8, while the long-term cytotoxicity experiments indicated that the liposomal preparation is better tolerated than aqueous PVP-iodine in the majority of cell lines tested. PVP-iodine in liposomal form is not genotoxic.

### Test III

A 3% PVP-iodine hydrogel liposomal preparation was compared with a 3% PVP-iodine ointment in which the active agent was not in liposomal form. The agent was applied to standardized *in vitro* cultures of rat skin and peritoneal explants, as a screening for tissue compatibility of skin and wound anti-infective agents.

The growth rate of the cultured explants was studied after 30 min exposure and incubation with a test substance.

Again, the results obtained show a substantially better toleration of the liposomal preparation in terms of peritoneum growth rate and skin growth rate.

With the ointment, the peritoneum growth rate reached 85%, and the skin growth rate reached 90%, while with the liposomal hydrogel formulation, the peritoneum growth rate was 96% and the skin growth rate 108%., respectively These values are to be compared with 100% values in a control test using Ringer's solution as the agent.

### Test IV

The toleration of liposomal PVP-iodine solutions for nasal applications was studied by investigating the influence of different test substances on ciliated epithelium cells, the most sensitive cells of the mucous membrane. A cytotoxic damage of these cells which would cause a restriction of the mucociliar clearance can be determined via a decrease of ciliary vibration.

Human ciliated epithelium cells were analyzed by an *in vitro* method which allows for determining ciliary activity or ciliary vibration. The corresponding cells were exposed and incubated with 100 µl test substance at a temperature of 37°C. After an incubation period of 5 min ciliary vibration was measured.

By using this *in vitro* method, a nutrient solution (Dulbeco) as a standard, a 0.2% chlorohexidine solution (a common antiseptic agent), conventional polyvidone iodine solutions (Betaisodona) of different concentrations (5.0%, 2.5%, and 1.25% PVP-iodine), and a liposomal solution containing 4.5% PVP-iodine were tested.

The results demonstrated that the cells tolerated the liposomal preparation substantially better: when the ciliated epithelium cells were exposed to the Betaisodona solutions containing 5.0% and 2.5% PVP-iodine, respectively, no ciliary activity could be observed after the incubation period. In contrast, treatment of the cells with a chlorohexidine solution led to a decrease of the measured ciliary vibration in comparison to the nutrient solution (i.e. the control). The low concentrated Betaisodona solution containing 1.25% PVP-iodine did not cause a detectable decrease of the ciliary activity. With respect to the measured ciliary vibration no difference to the control could be determined by exposing the human ciliated epithelium cells to the concentrated liposomal 4.5% PVP-iodine solution.

These results indicate that the liposomal formulation is well tolerated for nasal application and advantageous over chlorohexidine or conventional Betaisodona solutions.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Pharmaceutical composition for topical application, comprising as active ingredients:
(a) at least one antiseptic agent and/or at least one agent promoting wound healing;
and
(b) L-arginine.

2. The pharmaceutical composition of claim 1, wherein the composition is a hydrosome composition or a liposome composition.

3. The pharmaceutical composition of claim 1 or 2, wherein the final concentration of L-arginine is in the range of 1 mM to 1.000 mM, preferably in the range of 3 mM and 800 mM, and most preferably in the range of 5 mM and 600 mM.

4. The pharmaceutical composition of any of claims 1 to 3, wherein the antiseptic agent is selected from the group consisting of metal compounds, phenol derivatives, detergents, iodine, and iodine complexes.

5. The pharmaceutical composition of claim 4, wherein the antiseptic agent is povidone iodine.

6. The pharmaceutical composition of any of claims 1 to 5, wherein the wound healing promoting agent is selected from the group consisting of allantoin, an azulene compound, and a compound from the vitamin B group.

7. The pharmaceutical composition of any of claims 1 to 6, wherein the composition is a liposome composition, the liposomes having a substantially uniform size in the range between 20 nm and 20.000 nm, preferably in the range between 50 nm and 4.000 nm, and most preferably in the range between 500 nm and 2.500 nm.

8. The pharmaceutical composition of any of claims 1 to 7, wherein the composition releases the active ingredients over an extended time period.

9. The pharmaceutical composition of claim 8, wherein the composition releases the active ingredients at substantially the same release rate over the release time period.

10. The pharmaceutical composition of any of claims 1 to 9, further comprising at least one anaesthetic agent.

11. The pharmaceutical composition of any of claims 1 to 10, wherein the composition is provided in a dosage form selected from the group consisting of solutions and dispersions.

12. The pharmaceutical composition of any of claims 1 to 10, wherein the composition is provided in a dosage form selected from the group consisting of oil-in-water emulsions and water-in-oil emulsions.

13. The pharmaceutical composition of claim 12, wherein the emulsions are selected from the group consisting of ointments, creams, lotions, and balms.

14. The pharmaceutical composition of any of claims 1 to 10, wherein the composition is provided in the dosage form of a pharmaceutical gel, preferably a hydrogel, and most preferably a non-alcoholic hydrogel.

15. Use of a pharmaceutical composition of any of claims 1 to 14 for the manufacture of a medicament for the treatment of wounds.
